(19) Europäisches Patentamt
European Patent Office
Office européen des brevets

(11) EP 3 504 978 A1

(12) EUROPEAN PATENT APPLICATION
published in accordance with Art. 153(4) EPC

(43) Date of publication:
03.07.2019 Bulletin 2019/27

(51) Int Cl.:
A23C 9/12 (2006.01)      A23L 29/00 (2016.01)
A23P 10/35 (2016.01)     A23P 10/20 (2016.01)
A23P 10/40 (2016.01)

(21) Application number: 16914289.0

(22) Date of filing: 20.09.2016

(86) International application number:
PCT/KR2016/010474

(87) International publication number:
WO 2018/038310 (01.03.2018 Gazette 2018/09)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
MA MD

(30) Priority: 25.08.2016 KR 20160108466

(71) Applicant: C&M Tech Co., Ltd.
Gwangju-si, Gyeonggi-do 12748 (KR)

(72) Inventors:
• KWAK, Hae-Soo
Seoul 05371 (KR)
• AHN, Sung-Il
Seoul 05057 (KR)

(74) Representative: Byrne, Declan
Andre Roland S.A.
Intellectual Property Services
P.O. Box 5107
1002 Lausanne (CH)

(54) LACTASE-CONTAINING DOUBLE MICROCAPSULE, PREPARATION METHOD THEREFOR, AND USE THEREOF

(57) The present invention provides a lactase-containing double microcapsule which includes: lactase provided as a core material; and a primary coating material and a secondary coating material, which are sequentially coated on the core material to be formulated, a method for preparing the lactase-containing double microcapsule, and a dairy product including the lactase-containing double microcapsule as a use of the lactase-containing double microcapsule.

[FIG. 12]

EP 3 504 978 A1

**Description**

[Technical Field]

The present invention relates to a lactase-containing double microcapsule, a preparation method therefor, and a use thereof, and more particularly, to a lactase-containing double microcapsule which includes: lactase provided as a core material; and a primary coating material and a secondary coating material, which are sequentially coated on the core material to be formulated, a method for preparing the lactase-containing double microcapsule, and a dairy product including the lactase-containing double microcapsule as a use of the lactase-containing double microcapsule.

[Background Art]

Humans have been using milk as a food resource since about 8000 B.C. However, more than two-thirds of the world's population cannot digest milk properly. This is due to the fact that a sufficient amount of lactase is not secreted into a human body, which is referred to as lactose intolerance.

Lactase is an enzyme secreted from a small intestine, and serves to digest and absorb the milk by decomposing lactose, which is a disaccharide, into glucose and galactose, which are monosaccharides. When a person having lactose intolerance drinks milk, symptoms such as abdominal pain, diarrhea, nausea, abdominal bloating, and the like may occur.

In order to solve the above-described problems, an enzyme immobilization technique, membrane filtration technique using an ultrafiltration (UF)/reverse osmosis (RO) system, microencapsulation technique, and the like have been developed in the art.

The enzyme immobilization technique may continuously treat a large amount of milk with a small amount of enzyme. However, as the lactose is decomposed into glucose and galactose, a sugar content of milk is increased about four times, thereby resulting in consumers avoiding drinking the milk.

The membrane filtration technique using the UF/RO system is a technique that can selectively remove only the lactose from the milk. However, this technique causes a loss in a yield of about 5% during a process of treating the milk, thereby resulting in a deterioration in the intrinsic sweetness of milk due to an elimination of the lactose.

More seriously, when drinking lactose-free milk, a rate of transferring calcium contained in the milk to the bone is reduced (Kwak et al., Int. Dairy J. 22, 147-151 (2012)). In addition, costs of the apparatus and maintenance are expensive, thereby contributing to increasing a price of the product.

The lactase microencapsulation technique is a technique to add microencapsulated lactase to milk without performing any physical or chemical treatment, and does not cause a deterioration in an inherent taste of milk while having simple and economical processes. However, the existing water-in-oil (W/O) emulsion type microcapsule is very disadvantageous for long-term storage, since it may be easily oxidized in the air and is difficult to be pulverized because an outer layer thereof is formed as an oil layer.

A water-in-oil-in-water (W/O/W) emulsion type microcapsule includes water-soluble whey protein isolate (WPI) or maltodextrin used as a material of the outer layer, such that it is easily pulverized and is advantageous for long-term storage and treatment. However, when adding the above component to milk, there is a disadvantage that a stability in the milk is lowered.

Lactase microencapsulation using an enteric coating material is a method that may greatly improve not only the above-described advantage of the microencapsulation but also the stability in the milk, due to using the enteric coating material which is not dissolved in a gastric fluid and is dissolved only in the small intestine.

Nevertheless, as a conventional technique for microencapsulation of lactase, there only have been known a method for preparing milk containing lactase microencapsulated with lipid (Korean Patent Laid-Open Publication No. 10-1993-0022957), and a method for preparing lactase microencapsulated with lipid for addition to milk (Korean Patent Laid-Open Publication No. 10-1993-0022958). However, research on the lactase microencapsulation using the enteric coating material has not been conducted yet.

Moreover, in recent years, as rapidly moving toward an aged society, interest in the health of the elderly is increasing, and therefore developments of the above-described techniques are urgently required.

[Summary of Invention]

[Problems to be Solved by Invention]

In consideration of the above-described circumstances, it is an object of the present invention to provide a lactase-containing double microcapsule for solving lactose intolerance of dairy products.

In addition, another object of the present invention is to provide a method for preparing the lactase-containing double microcapsule.

Further, another object of the present invention to provide a dairy product including the lactase-containing double microcapsule.

[Means for Solving Problems]

In order to achieve the above objects, according to an aspect of the present invention, there is provided a lactase-containing double microcapsule including: lactase provided as a core material; and a primary coating material and a secondary coating material, which are sequentially coated on the core material to be formulated.

According to another aspect of the present invention, there is provided a method for preparing lactase including: providing lactase as a core material, and stirring the core material with a primary coating material and a primary emulsifier to obtain an emulsion of lactase; stirring the emulsion of lactase with a secondary coating material and a secondary emulsifier to obtain a double microcapsule emulsion of lactase; and formulating the obtained double microcapsule emulsion of lactase.

In addition, according to another aspect of the present invention, there is provided a dairy product including the lactase-containing double microcapsule.

[Advantageous Effects]

According to the present invention, it is possible to provide a lactase-containing double microcapsule formulated by providing lactase as the core material, and sequentially coating the core material with the primary coating material and the secondary coating material to be formulated.

Meanwhile, since the lactase-containing double microcapsule according to the present invention uses an enteric coating material which is dissolved only in the small intestine, it is possible to greatly improve not only the advantage of the microencapsulation but also the stability in the milk, as well as solve the lactose intolerance of dairy products.

[Brief Description of Drawings]

FIG. 1 is a schematic view illustrating microencapsulation according to one embodiment.

FIG. 2 is a graph illustrating changes in an amount of absorbed moisture with an addition of medium-chain triglyceride (MCT).

FIG. 3 is a graph illustrating a reaction surface for microencapsulation yield of a enteric-coated lactase microcapsule.

FIG. 4 is a diagram illustrating an optimization curve in a process of the lactase microencapsulation, wherein y and d represent a yield of microcapsules and a statistically desirable score of the optimized condition, respectively.

FIG. 5 is photographs illustrating observation results of enteric lactase microcapsules using a microscope, wherein A, B, and C represent results of hydroxypropyl methylcellulose phthalate (HPMCP), shellac, and zein, respectively.

FIG. 6 is a graph illustrating zeta potential of the enteric lactase microcapsules.

FIG. 7 is a graph illustrating particle diameters of the enteric lactase microcapsules.

FIG. 8 is a graph illustrating physicochemical properties of milk added with enteric lactase microcapsules, in which the control is a milk product in the market.

FIG. 9 is a graph illustrating stabilities of lactase microcapsules.

FIG. 10 is a graph illustrating in vitro release characteristics of the enteric lactase microcapsules at pH 2, 3, and 4.

FIG. 11 is a graph illustrating in vitro release characteristics of the enteric lactase microcapsules at pH 6, 7, and 8.

FIG. 12 is a graph illustrating changes in blood glucose after drinking 500 mL of milk supplemented with HPMCP-coated lactase microcapsules.

FIG. 13 is a graph illustrating results of evaluating the symptom intensity of diarrhea after 5 minutes from drinking 500 mL of milk supplemented with HPMCP-coated lactase microcapsules by a 5-score method.

FIG. 14 is a graph illustrating results of evaluating the symptom intensity of abdominal pain by the 5-score method after drinking 500 mL of milk supplemented with HPMCP-coated lactase microcapsules.

FIG. 15 is a graph illustrating results of evaluating the symptom intensity of audible bowel sound by the 5-score method after drinking 500 mL of milk supplemented with HPMCP-coated lactase microcapsules.

FIG. 16 is a graph illustrating results of evaluating the symptom intensity of abdominal flatulence by the 5-score method after drinking 500 mL of milk supplemented with HPMCP-coated lactase microcapsules.

FIG. 17 is a graph illustrating results of evaluating the symptom intensity of nausea by the 5-score method after drinking 500 mL of milk supplemented with HPMCP-coated lactase microcapsules.

[Mode for Carrying out Invention]

The present invention discloses a lactase-containing double microcapsule including: lactase provided as a core material; and a primary coating material and a secondary coating material, which are sequentially coated on the core material to be formulated.

In the lactase-containing double microcapsule of the present invention, the lactase, which is the core material of the lactase-containing double microcapsules, may be derived from a strain selected from the group consisting of *Kluyveromyces lactis, Aspergillus oryzae, Aspergillus niger, Bacillus circulans, Escherichia coli* and *Bos Taurus,* or may be recombinant lactase.

In the lactase-containing double microcapsule of the present invention, the primary coating material to be used may include medium-chain triglyceride (MCT).

In the lactase-containing double microcapsule of the present invention, the primary coating material to be used may include hydrogenated corn oil.

In the lactase-containing double microcapsule of the present invention, the primary coating material to be used may include soybean oil.

In the lactase-containing double microcapsule of the present invention, the primary coating material to be used may include safflower seed oil.

In the lactase-containing double microcapsule of the present invention, the primary coating material to be used may include butter oil.

In the lactase-containing double microcapsule of the present invention, the primary coating material to be used may include at least two selected from the group consisting of MCT, hydrogenated corn oil, soybean oil, safflower seed oil and butter oil.

In the lactase-containing double microcapsule of the present invention, the primary coating material to be used may include any material suitable for edible purposes.

In the lactase-containing double microcapsule of the present invention, the secondary coating material to be used may include hydroxypropyl methylcellulose phthalate (HPMCP) .

In the lactase-containing double microcapsule of the present invention, the secondary coating material to be used may include zein.

In the lactase-containing double microcapsule of the present invention, the secondary coating material to be used may include shellac.

In the lactase-containing double microcapsule of the present invention, the secondary coating material to be used may include at least one selected from the group consisting of hydroxypropyl methylcellulose phthalate, zein, shellac, Eudragit, cellulose acetate phthalate, cellulose acetate succinate, polyvinyl acetate phthalate, cellulose acetate trimellitate, hypromellose acetate succinate and phenyl salicylate.

In the lactase-containing double microcapsule of the present invention, examples of the Eudragit may include Eudragit L-100, Eudragit S-100 and the like.

In the lactase-containing double microcapsule of the present invention, the secondary coating material to be used may include Eudragit.

In the lactase-containing double microcapsule of the present invention, the secondary coating material to be used may include any material suitable for edible purposes.

In the lactase-containing double microcapsule of the present invention, the double microcapsule may have a particle diameter of several nm to several mm.

In the lactase-containing double microcapsule of the present invention, the double microcapsule may have a particle diameter of 100 nm to 1 mm.

In the lactase-containing double microcapsule of the present invention, the double microcapsule may have a particle diameter of 1 to 10 $\mu$m.

In the lactase-containing double microcapsule of the present invention, the formulation may be in any form selected from the group consisting of powders, solution, tablets, and granules.

In the lactase-containing double microcapsule of the present invention, the microcapsule may be lactase-containing double microcapsule powders which include: lactase provided as a core material; and a primary coating material and a secondary coating material, which are sequentially coated on the core material, and then pulverized.

In the lactase-containing double microcapsule of the present invention, the microcapsule may be lactase-containing double microcapsule powders which include: lactase provided as a core material; and a primary coating material and a secondary coating material, which are sequentially coated on the core material, and then formulated in a solution.

In the lactase-containing double microcapsule of the present invention, the microcapsule may be lactase-containing double microcapsule powders which include: lactase provided as a core material; and a primary coating material and a secondary coating material, which are sequentially coated on the core material, and then formulated in tablets.

In the lactase-containing double microcapsule of the present invention, the microcapsule may be lactase-containing double microcapsule powders which include: lactase provided as a core material; and a primary coating material and a secondary coating material, which are sequentially coated on the core material, and then formulated in granules.

In the lactase-containing double microcapsule of the present invention, the double microcapsule may further include a functional component in addition to the lactase as the core material.

In the lactase-containing double microcapsule of the present invention, when further including the functional component in addition to the lactase as the core material, a mixture, in which the lactase and the functional component are mixed in a weight ratio of 1:9 to 9:1, may be used.

In the lactase-containing double microcapsule of the present invention, the functional component to be used may include Juniperus rigida fruit extract, which is a fruit extract of Juniperus rigida S. et Z.

In the lactase-containing double microcapsule of the present invention, the Juniperus rigida fruit extract may be obtained by: putting and mixing Juniperus rigida fruit in purified water of 5 to 20 times based on a weight of the Juniperus rigida fruit; then extracting and filtering the same so as to have a volume of 10 to 50% based on a volume of initial purified water at a temperature of 90 to 120°C to obtain a primary filtrate; and decompression concentrating the primary filtrate, followed by vacuum freeze drying so as to have a volume of 10 to 50% based on a volume of the primary filtrate, thus to be used as the functional component.

In addition, the present invention discloses a method for preparing the lactase-containing double microcapsule.

The method for preparing a lactase-containing double microcapsule of the present invention may be a method for preparing a lactase-containing double microcapsule which includes: providing lactase as a core material, and stirring the core material with a primary coating material and a primary emulsifier to obtain an emulsion of lactase; stirring the emulsion of lactase with a secondary coating material and a secondary emulsifier dissolved in alcohol to obtain a double microcapsule emulsion of lactase; and pulverizing the double microcapsule emulsion of lactase.

In the step of obtaining the emulsion of lactase in the method for preparing a lactase-containing double microcapsule of the present invention, the primary coating material to be used for coating the core material may include medium-chain triglyceride (MCT).

In the step of obtaining the emulsion of lactase in the method for preparing a lactase-containing double microcapsule of the present invention, the primary coating material to be used for coating the core material may include hydrogenated corn oil.

In the step of obtaining the emulsion of lactase in the method for preparing a lactase-containing double microcapsule of the present invention, the primary coating material to be used for coating the core material may include soybean oil.

In the step of obtaining the emulsion of lactase in the method for preparing a lactase-containing double microcapsule of the present invention, the primary coating material to be used for coating the core material may include safflower seed oil.

In the step of obtaining the emulsion of lactase in the method for preparing a lactase-containing double microcapsule of the present invention, the primary coating material to be used for coating the core material may include butter oil.

In the step of obtaining the emulsion of lactase in the method for preparing a lactase-containing double microcapsule of the present invention, the primary coating material to be used for coating the core material may include at least two selected from the group consisting of MCT, hydrogenated corn oil, soybean oil, safflower seed oil and butter oil.

In the step of obtaining the emulsion of lactase in the method for preparing a lactase-containing double microcapsule of the present invention, the primary coating material to be used for coating the core material may include any material suitable for edible purposes.

In the step of obtaining the emulsion of lactase in the method for preparing a lactase-containing double microcapsule of the present invention, the primary emulsifier to be used may include polyglycerol polyricinoleate, sucrose fatty acid ester, and polyglycerol fatty acid ester.

In the method for preparing a lactase-containing double microcapsule of the present invention, the primary emulsifier may be used with a hydrophile-lipophile balance (HLB) value of 0.5 to 5.

In the method for preparing a lactase-containing double microcapsule of the present invention, the primary emulsifier may be used in a concentration of 0.25 to 1.25%.

In the method for preparing a lactase-containing double microcapsule of the present invention, the primary emulsifier may include polyglycerol polyricinoleate which is used with an HLB value of 0.5 to 5, and in a concentration of 0.25 to 1.25%.

In the method for preparing a lactase-containing double microcapsule of the present invention, the primary emulsifier may include sucrose fatty acid ester which is used with an HLB value of 0.5 to 5, and in a concentration of 0.25 to 1.25%.

In the method for preparing a lactase-containing double microcapsule of the present invention, the primary emulsifier may include polyglycerol fatty acid ester which is used with an HLB value of 0.5 to 5, and in a concentration of 0.25 to 1.25%.

In the step of obtaining the emulsion of lactase in the method for preparing a lactase-containing double microcapsule of the present invention, the emulsion of lactase may be obtained by stirring the core material, the primary coating material and the primary emulsifier to obtain a pre-emulsion, followed by stirring the same.

In the step of obtaining the lactase emulsion of lactase-containing double microcapsule of the present invention, the lactase emulsion may be obtained by stirring the core material, the primary coating material and the primary emulsifier at 35 to 40°C and 1,000 to 2,000 rpm for 20 to 40 minutes to obtain a pre-emulsion, followed by stirring the same at 35 to 40°C and 8,500 to 9,500 rpm for 1 to 3 minutes.

In the step of obtaining the lactase emulsion of lactase-containing double microcapsule of the present invention, the lactase emulsion may be obtained by stirring the core material, the primary coating material and the primary emulsifier at 37.5°C and 1,500 rpm for 30 minutes to obtain a pre-emulsion, followed by stirring the same at 37.5°C and 9,000 rpm for 2 minutes.

In the step of obtaining the lactase emulsion of the method for preparing a lactase-containing double microcapsule of the present invention, the core material and the primary coating material may be mixed in a ratio of 1:9 (v/v) to 9:1 (v/v), and then stirred.

In the step of obtaining the lactase emulsion of the method for preparing a lactase-containing double microcapsule of the present invention, the core material and the primary emulsifier may be mixed in a ratio of 1:9 (v/v) to 9:1 (v/v), and then stirred.

In the method for preparing a lactase-containing double microcapsule of the present invention, when preparing the double microcapsule emulsion of lactase, the secondary coating material to be used may include hydroxypropyl methylcellulose phthalate (HPMCP).

In the method for preparing a lactase-containing double microcapsule of the present invention, the secondary coating material to be used may include zein.

In the method for preparing a lactase-containing double microcapsule of the present invention, the secondary coating material to be used may include shellac.

In the method for preparing a lactase-containing double microcapsule of the present invention, the secondary coating material to be used may include at least one selected from the group consisting of hydroxypropyl methylcellulose phthalate, zein, shellac, Eudragit, cellulose acetate phthalate, cellulose acetate succinate, polyvinyl acetate phthalate, cellulose acetate trimellitate, hypromellose acetate succinate and phenyl salicylate.

In the method for preparing a lactase-containing double microcapsule of the present invention, examples of the Eudragit may include Eudragit L-100, Eudragit S-100 and the like.

In the method for preparing a lactase-containing double microcapsule of the present invention, the secondary coating material to be used may include Eudragit.

In the method for preparing a lactase-containing double microcapsule of the present invention, when preparing the double microcapsule emulsion of lactase, the secondary coating material to be used may include any material suitable for edible purposes.

In the method for preparing a lactase-containing double microcapsule of the present invention, the alcohol may be alcohol having 1 to 4 carbon atoms, and ethanol is preferably used.

In the method for preparing a lactase-containing double microcapsule of the present invention, when preparing the double microcapsule emulsion of lactase, the secondary emulsifier to be used may include Tween 60.

In the method for preparing a lactase-containing double microcapsule of the present invention, when preparing the double microcapsule emulsion of lactase, the secondary emulsifier to be used may include polyoxyethylene sorbitan monolaurate (PSML).

In the method for preparing a lactase-containing double microcapsule of the present invention, the secondary emulsifier may be used with a hydrophile-lipophile balance (HLB) value of 13 to 17.

In the method for preparing a lactase-containing double microcapsule of the present invention, the secondary emulsifier may be used in a concentration of 0.5 to 1.5%.

In the method for preparing a lactase-containing double microcapsule of the present invention, the secondary emulsifier may include polyoxyethylene sorbitan monolaurate (PSML) which is used with an HLB value of 13 to 17, and in a concentration of 0.5 to 1.5%.

In the method for preparing a lactase-containing double microcapsule of the present invention, the secondary emulsifier may include Tween 60 which is used with an HLB value of 13 to 17, and in a concentration of 0.5 to 1.5%.

In the step of obtaining the double microcapsule emulsion of lactase in the method for preparing a lactase-containing double microcapsule of the present invention, the double microcapsule emulsion of lactase may be obtained by stirring the emulsion of lactase, the secondary coating material and the secondary emulsifier to homogenize the same, followed by centrifuging the same.

In the step of obtaining the double microcapsule emulsion of lactase in the method for preparing a lactase-containing double microcapsule of the present invention,
the double microcapsule emulsion of lactase may be obtained by stirring the emulsion of lactase, the secondary coating material and the secondary emulsifier at 35 to 40°C and 1500 to 2,500 rpm for 5 to 30 minutes to homogenize the same, followed by centrifuging at 3 to 5°C and 3,000 xg to 4,000 xg for 10 to 20 minutes.

In the step of obtaining the double microcapsule emulsion of lactase in the method for preparing a lactase-containing double microcapsule of the present invention, the emulsion of lactase and the secondary coating material may be mixed in a ratio of 1:9 (v/v) to 9:1 (v/v), and then stirred, followed by centrifuging to obtain a double microcapsule emulsion of lactase.

In the step of obtaining the double microcapsule emulsion of lactase in the method for preparing a lactase-containing double microcapsule of the present invention, the emulsion of lactase and the secondary emulsifier may be mixed in a ratio of 1:9 (v/v) to 9:1 (v/v), and then stirred, followed by centrifuging to obtain a double microcapsule emulsion of lactase.

As an example of the method for preparing a lactase-containing double microcapsule of the present invention, the lactase-containing double microcapsule may be prepared by comprising: providing lactase as a core material, and stirring the core material with medium-chain triglyceride (MCT) as a primary coating material and at least one selected from the group consisting of polyglycerol polyricinoleate, sucrose fatty acid ester, and polyglycerol fatty acid ester as a primary emulsifier to obtain a pre-emulsion, and further stirring the mixture to obtain an emulsion of lactase, wherein the core material and the primary coating material are mixed in a ratio of 1:9 (v/v) to 9:1 (v/v), and the core material and the primary emulsifier are mixed in a ratio of 1:9 (v/v) to 9:1 (v/v), and then stirred to obtain an emulsion of lactase; providing the emulsion of lactase as a core material, adding any one selected from the group consisting of hydroxypropyl methylcellulose phthalate, zein, and shellac as a secondary coating material, and any one selected from the group consisting of Tween 60 and polyoxyethylene sorbitan monolaurate as a secondary emulsifier to the obtained emulsion of lactase, and stirring to homogenize the same, followed by centrifuging to obtain a double microcapsule emulsion of lactase, wherein the emulsion of lactase and the secondary coating material are mixed in a ratio of 1:9 (v/v) to 9:1 (v/v), and the emulsion of lactase and the secondary emulsifier are mixed in a ratio of 1:9 (v/v) to 9:1 (v/v), and then stirred, followed by centrifuging to obtain a double microcapsule emulsion of lactase; and formulating the obtained double microcapsule emulsion of lactase.

As another example of the method for preparing a lactase-containing double microcapsule of the present invention, the lactase-containing double microcapsule may be prepared by comprising: providing lactase as a core material, and stirring the core material with MCT as a primary coating material and at least one selected from the group consisting of polyglycerol polyricinoleate, sucrose fatty acid ester, and polyglycerol fatty acid ester as a primary emulsifier at 35 to 40°C and 1,000 to 2,000 rpm for 20 to 40 minutes to obtain a pre-emulsion, followed by stirring the same at 35 to 40°C and 8,500 to 9,500 rpm for 1 to 3 minutes to obtain an emulsion of lactase, wherein the core material and the primary coating material are mixed in a ratio of 1:9 (v/v) to 9:1 (v/v), and the core material and the primary emulsifier are mixed in a ratio of 1:9 (v/v) to 9:1 (v/v), and then stirred to obtain an emulsion of lactase; providing the emulsion of lactase as a core material, adding any one selected from the group consisting of hydroxypropyl methylcellulose phthalate, zein and shellac as a secondary coating material, and any one selected from the group consisting of Tween 60 and polyoxyethylene sorbitan monolaurate as a secondary emulsifier to the obtained emulsion of lactase, and stirring to homogenize the same at 35 to 40°C and 1,500 to 2,500 rpm for 5 to 30 minutes to homogenize the same, followed by centrifuging at 3 to 5°C and 3,000 xg to 4,000 xg for 10 to 20 minutes to obtain a double microcapsule emulsion of lactase, wherein the emulsion of lactase and the secondary coating material are mixed in a ratio of 1:9 (v/v) to 9:1 (v/v), and the emulsion of lactase and the secondary emulsifier are mixed in a ratio of 1:9 (v/v) to 9:1 (v/v), and then stirred, followed by centrifuging to obtain a double microcapsule emulsion of lactase; and formulating the obtained double microcapsule emulsion of lactase.

In the method for preparing a lactase-containing double microcapsule of the present invention, the step of formulating the obtained double microcapsule emulsion of lactase may use a method capable of formulating the double microcapsule emulsion of lactase which is a liquid phase in food industrial fields.

In the method for preparing a lactase-containing double microcapsule of the present invention, the formulation may be in any form selected from the group consisting of powders, solution, tablets, and granules.

In the method for preparing a lactase-containing double microcapsule of the present invention, as an example of the method for formulating the obtained double microcapsule emulsion of lactase, at least one method selected from the group consisting of spray chilling, spray cooling, spray drying, suspension processing, extrusion, coacervation, cocrystallization, molecular inclusion, freeze drying, and rotational suspension separation, which are pulverization methods, may be used.

In the method for preparing a lactase-containing double microcapsule of the present invention, the spray drying is the most typical method, which is economically advantageous for industrialization and is used for encapsulating materials to be applied to foods. The spray drying is a method including hydrating a coating material, then dispersing a target material, and spraying a mixture thereof into a high-temperature chamber. That is, a coating material solution is micro-granulated by a spraying system, such that it comes into contact with hot air while a total specific surface area thereof is increased, and is rapidly evaporated to form double microcapsules of powders. Efficiency of the double microencapsulation by the spray drying depends on a form of the coating material used for encapsulation, which also affects emulsification stabilities of the double microcapsules before drying, and physical stabilities and a storage life thereof after drying. In addition, by performing pulverization, it is possible to increase preservation and treatment convenience of the double microcapsules.

When preparing the lactase-containing double microcapsule of the present invention, the double microcapsule may further include a functional component in addition to the lactase as the core material.

In the method for preparing a lactase-containing double microcapsule of the present invention, when further including the functional component in addition to the lactase as the core material, a mixture, in which the lactase and the functional component are mixed in a weight ratio of 1:9 to 9:1, may be used.

In the method for preparing a lactase-containing double microcapsule of the present invention, the functional component to be used may include Juniperus rigida fruit extract, which is a fruit extract of Juniperus rigida S. et Z.

In the method for preparing a lactase-containing double microcapsule of the present invention, the Juniperus rigida fruit extract may be obtained by: putting and mixing Juniperus rigida fruit in purified water of 5 to 20 times based on a weight of the Juniperus rigida fruit; then extracting and filtering the same so as to have a volume of 10 to 50% based on a volume of initial purified water at a temperature of 90 to 120°C to obtain a primary filtrate; and decompression concentrating the primary filtrate, followed by vacuum freeze drying so as to have a volume of 10 to 50% based on a volume of the primary filtrate, thus to be used as the functional component.

In addition to the above-described lactase-containing double microcapsule, the present invention further discloses a dairy product including the lactase-containing double microcapsule prepared by the above-described method.

The dairy product including the lactase-containing double microcapsule of the present invention may include the lactase-containing double microcapsule in an amount of 0.1 to 10% by weight based on the total weight of the dairy product.

In the dairy product including the lactase-containing double microcapsule of the present invention, the dairy product may be any one selected from the group consisting of milk, yogurt, goat milk, whey protein concentrates and ice cream.

In the dairy product including the lactase-containing double microcapsule of the present invention, the dairy product may be milk.

In the dairy product including the lactase-containing double microcapsule of the present invention, the dairy product may be yogurt.

In the dairy product including the lactase-containing double microcapsule of the present invention, the dairy product may be goat milk.

In the dairy product including the lactase-containing double microcapsule of the present invention, the dairy product may be whey protein concentrates.

In the dairy product including the lactase-containing double microcapsule of the present invention, the whey protein concentrates may be added to a health supplement for preventing diarrhea.

In the dairy product including the lactase-containing double microcapsule of the present invention, the dairy product may be ice cream.

The present inventors conducted experiments under various conditions for the lactase-containing double microcapsule of the present invention, the preparation method therefor and the use thereof, and the present invention has been completed on the basis of the finding that the lactase-containing double microcapsule, the preparation method therefor and the use thereof are provided by the above-described conditions, in order to achieve the objects of the present invention.

Hereinafter, the present invention will be described in detail with reference to the following experimental examples, examples and application examples. However, these examples are intended to describe the present invention in more detail, and the scope of the present invention is not limited thereto.

<Experimental Example> Preparation of double microcapsule of lactase

I. Materials and methods

1. Materials and reagents

1) Materials

(1). Core material

Godo YNL-2 (Godo Shusei Co., Ltd., Tokyo, Japan) was used as lactase. This enzyme is derived from *Kluyveromyces lactis* and has an activity of 7,448 units/g. Herein, 1 unit is defined in such a manner that 1 $\mu$mol o-nitrophenyl $\beta$-D-galactopyranoside (ONPG) is decomposed into o-nitrophenol and D-galactose per minute.

(2). Coating material

Medium-chain triglyceride (MCT), which is a primary coating material, was purchased from Wellga Co., Ltd. (Seongnam, Korea). HPMCP, shellac, and zein as secondary coating materials were purchased from Samsung Fine Chemicals Co., Ltd., Shellac Korea Co., Ltd., and Richwood Trading (Pooglimmuyark) Co., Ltd.

(3). Emulsifier

Polyglycerol polyricinoleate (PGPR; HLB 0.6) and polyoxyethylene sorbitan monolaurate (PSML; HLB 16.7), which are food additive grade products with a purity of 95.0% or higher, were purchased from IlshinWells (Seoul, Korea).

2. Experimental Method

1) Preparation of double microcapsule powder of lactase

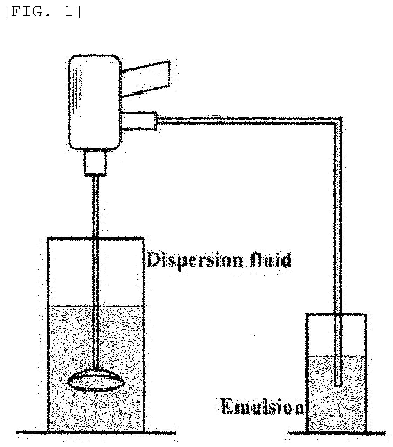

Microencapsulation of lactase using an enteric coating material was performed by modifying a method of Quispe-Condori et al. (2011). Lactase was added with MCT, which is a primary coating material and serves as a plasticizer of the enteric coating material, and PGPR 0.75% (w/v) as an emulsion, then the mixture was homogenized by a high speed homogenizer (HMZ-20DN, Poonglim Co., Seoul, Korea) at a rate of 9,000 rpm for 1 minute.
As the schematic view of microencapsulation illustrated in FIG. 1, HPMCP as an enteric secondary coating material was prepared by dissolving in 85% (v/v) of ethanol, followed by mixing the primary emulsion therein to homogenize at 2,500 rpm for 4 minutes, then the mixture was sprayed into a cold dispersion liquid containing 0.25% of PSML using an airless spray gun (W-300, Wagner GmbH, Markdorf, Germany).
The microcapsules were separated by performing centrifugation at 2,500 rpm for 4 minutes. Then, the microcapsules were washed three times and frozen at -80°C using a deep freezer (MDF-U53V, Sanyo Denki Co., Ltd., Tokyo, Japan), and were freeze dried, followed by performing pulverization.

2) . Experimental design using response surface analysis

Process parameters that affect the microencapsulation process were set to be a coating material $X_1$ and a core material $X_2$, while being set in a range of 1 to 15 g/100 mL, and 3 to 15 mL, respectively. Results thereof are coded in three levels of -1, 0 and +1, and are stated in Table 1 below.

[Table 1]

| Variables | Coded $X_i$ | Coded level | | |
|---|---|---|---|---|
| | | -1 | 0 | +1 |
| Concentration of coating material (g/100mL) | $X_1$ | 1 | 8 | 15 |
| Amount of core material (mL) | $X_2$ | 3 | 9 | 15 |

An experiment was performed on a total of 11 experimental groups according to a central composite design. Results thereof were statistically processed with Minitab version 16, and summarized as Equation 1 below. In the following Equation 1, Y is a dependent variable, and $\beta_0$, $\beta_1$, and $\beta_2$ are constants of each term.

[Equation 1]

$$Y_n = \beta_0 + \beta_1 x_1 + \beta_2 x_2 + \beta_{12} x_1 x_2 - \beta_{11} x_1^2 + \beta_{12} x_2^2$$

3). Microencapsulation yield

In order to measure the microencapsulation yield of lactase, a method of Kawaktsu et al. Colloids Surf. A, 189, 257-264 (2001) was applied to the measurement with some modification.

To separate the unencapsulated filtrate from the double coated emulsion, centrifugation was performed at a rate of 3,000 xg for 5 minutes. The filtrate separated by centrifugation was filtered by using a syringe filter with a 0.45 $\mu$m pore size.

2 mL of 5 mM ONPG solution preheated at 37°C for 15 minutes in a water bath was added to 0.5 mL of the sample obtained as described above, and a reaction was performed at 37°C for 15 minutes. The reaction was stopped by adding 0.5 mL of 500 mM sodium carbonate solution to the mixture with cooling, and absorbances thereof at 420 nm were measured.

The microencapsulation yield was calculated using Equation 2 below, and the sample measurement was repeated three times.

[Equation 2]

$$Microencapsulation\ yield\ (\%) = 1 - \frac{activity\ of\ uncapsulated\ part}{initial\ activity\ of\ lactase} \times 100$$

4). Microscopic observation of lactase microcapsules

By the optimized process using RSM, the lactase microcapsules coated with the secondary coating material of HPMCP, shellac, and zein were observed using an optical microscope (Eclipse 80i, Nikon, Tokyo, Japan) by magnifying the same to 1,000 times.

5). Scanning electron microscope (SEM) observation of lactase microcapsule powder

Lactase microcapsule powders were observed by SEM in order to determine surface characteristics thereof.

6). Analysis of particle size

Analysis of particle sizes of the lactase microcapsule powders was performed using a particle diameter analyzer (Mastersizer 2000, Malvern Instruments Ltd., Worcestershire, UK). Using an optimal condition, lactase microcapsules coated with various secondary coating materials were added to 10 mL of distilled water in a ratio of 1:400 (w/v), and subjected to measurement at 25°C.

7). Measurement of zeta potential

To estimate distributions of lactase microcapsules when dispersing in milk, zeta potential was measured using a particle diameter analyzer (ELSZ2-PLUS, Otsuka Electronics Co., Ltd., Osaka, Japan).

0.01 g of the sample was dispersed in 10 mL of distilled water adjusted to pH 2 to 12, and subjected to ultrasonification for 5 minutes, then placed at 25°C for 15 minutes. 1 mL of supernatant was taken and injected into a flow cell, and subjected to measurement of zeta potential at 25°C. At this time, the measurement points were 0.7, 0.35, 0, -0.35, and -0.7, respectively.

8). Evaluation of *in vitro* release characteristics of enteric lactase microcapsule

*In vitro* release characteristics of enteric lactase microcapsules were measured using artificial gastric fluid and artificial small intestinal fluid. Measurement of release characteristics in an artificial gastrointestinal environment was performed by modifying methods of Luan et al. Int. J. Pharm. 324, 168-175 (2006), and Papagianni et al. Enzyme Microb. Technol. 45, 514-552 (2009).

1 g of pepsin was dissolved in 25 mL of 0.1 M hydrochloric acid to prepare an artificial gastric fluid. 1 g of enteric microcapsules was dissolved in 100 mL of 5% (w/v) lactose solution, adjusted to pH 2, 3, and 4 using 6 M hydrochloric acid, and 1 mL of artificial gastric fluid was added thereto.

A degradation rate of enteric lactase microcapsules was measured by taking samples at an interval of 30 minutes while decomposing the same using a shaking water bath at 37°C and 100 rpm for 2 hours.

The degradation rate of enteric lactase microcapsules was measured by analyzing a content of the residual lactose. The content analysis of the residual lactose was performed by slightly modifying a method of Kwak & Jeon, J. Food Sci. 53, 975-976 (1988).

5 mL of sample and 25 mL of tertiary distilled water were mixed and placed in a 50 mL volumetric flask and diluted with acetonitrile to set a volume thereof. After mixing, centrifugation was performed at 5,000 xg for 10 minutes, and the supernatant was taken and filtered using a polyvinylidene fluoride (PVDF) filter with a 0.45 μm pore size.

The sample was analyzed using HPLC (Dionex Co., Sunnyvale, CA, USA) and a refractive index detector (RI-101, Showa Denko K. K. Co., Tokyo, Japan). In this case, $NH_2$ column (4.6 × 250 mm, Hector-M, RS tech Co., Daejeon, Korea) was used as a column, and the mobile phase was fed by flowing 75% (v/v) of acetonitrile at a rate of 2.0 mL/min.

9). Evaluation of physicochemical properties of milk added with enteric lactase microcapsules

In order to evaluate the physicochemical properties of milk added with enteric lactase microcapsules, the enteric microcapsules were added to 200 mL of market milk at 1% (w/v) and stored in an environment at 4°C for 12 days to observe the PH, chromaticity, and release rate of lactase.

At this time, the release rate of lactase was measured by content analysis of the residual lactose as described above.

10). Sensory evaluation

For the sensory evaluation of milk added with enteric lactase microcapsules, 10 male and female test panels (3 males and 7 females) who do not have objection to drink milk were selected, whose average age was 26 years.

The selected test panels were subjected to training for sweetness and off-taste three times for every 1 hour. 1% (w/v) of lactase microcapsules were added to the market milk, and used for performing the sensory test while storing in an environment at 4°C for 12 days.

The sample was put in a 50 mL of cup with a lid and provided to the test panels by marking an arbitrary 4-digit number. Sweetness and off-taste were represented using a 5-score method (1 = very weak, 3 = moderate, 5 = very strong).

11). Clinical test

To determine effects of improving lactose intolerance of milk applied with lactase microcapsules, first 20 patients having lactose intolerance (12 males and 8 females, whose average age was 41.7 years) were selected.

These patients had no history of gastrointestinal tract problems and no history of diabetes. After drinking 500 mL of market milk (corresponding to 25 g lactose), when blood glucoses of patients are increased to less than 20 mg/dL within 30 to 60 minutes, the patients are determined to have lactose intolerance (Finkenstedt et al., Brit. med. J. 292, 161-162, (1986); Greenberger & Isselbacher, harrison's principles of internal medicine, New York: McGraw-Hill, 1735 (1983)).

The test panels selected as described above drunk 500 mL of market milk mixed with 1 g of microcapsules on an empty stomach, then blood glucoses thereof were measured every 30 minutes for 3 hours.

At the same time, intensities of feeling various symptoms (diarrhea, abdominal pain, abdominal bloating, audible bowel sound, and nausea) caused by lactose intolerance were recorded by the test panels using the 5-score method (1 = none, 3 = moderate, 5 = extreme).

12). Statistical processing

Analysis of variance (ANOVA) was conducted on data obtained from the experiment using a statistical analysis system (SAS) 9.0 and a significance test was performed at a 5% significance level using least significant difference (LSD).

3. Results

1). Optimization of added amount of MCT

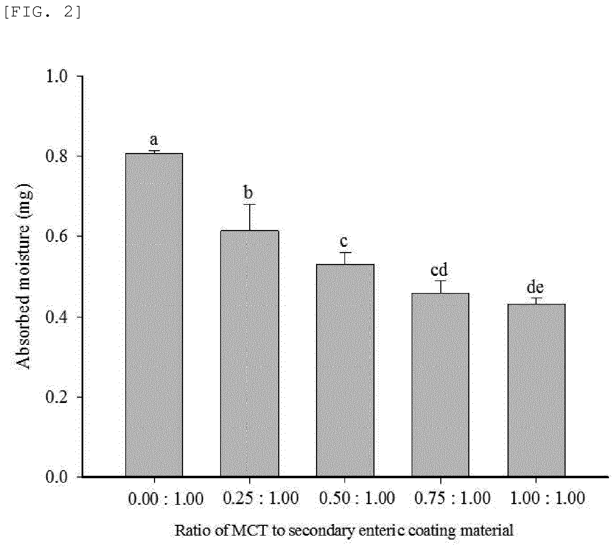

Changes in an amount of absorbed moisture with an addition of MCT are illustrated in FIG. 2. As illustrated in

**EP 3 504 978 A1**

FIG. 2, the amount of absorbed moisture tended to be decreased as a ratio of the MCT is increased. However, a significant difference was not observed from a ratio of 0.50:1.00 between the MCT and the enteric coating material.

Accordingly, it could be seen that a ratio of the added amount of MCT serving as the primary coating material to the plasticizer of the enteric coating material was most preferably 0.50:1.00.

2). Optimization of microencapsulation process using RSM

An experimental analysis was performed on a total of 11 experimental groups under a condition based on the central composite design, and results of measuring yields obtained from the respective experimental groups are stated in Table 2 below. Then, statistical processing was performed on the obtained results using Minitab 16 (Table 3).

[Table 2]

| Run number | Coded variable[1] | | Process variable | | Response variables[2] |
|---|---|---|---|---|---|
| | $X_1$ | $X_2$ | $X_1$ | $X_2$ | $Y$ |
| 1 | +1 | +1 | 15 | 15 | 99.7897 |
| 2 | 0 | 0 | 8 | 9 | 99.8787 |
| 3 | -1 | 0 | 1 | 9 | 81.4507 |
| 4 | +1 | -1 | 15 | 3 | 99.9912 |
| 5 | -1 | -1 | 1 | 3 | 98.2869 |
| 6 | 0 | 1 | 8 | 15 | 87.6300 |
| 7 | +1 | 0 | 15 | 9 | 99.9747 |
| 8 | -1 | +1 | 1 | 15 | 85.1009 |
| 9 | 0 | 0 | 8 | 9 | 99.9110 |
| 10 | 0 | 0 | 8 | 9 | 99.9435 |
| 11 | 0 | -1 | 8 | 3 | 99.9868 |

[1] $X_1$, and $X_2$ are the concentration of coating material (g/100mL) and the amount of β-galactosidase (mL), respectively.
[2] $Y$ is the yield of microencapsulation.

12

[Table 3]

| Independent variable[1] | Regression coefficient | Standard error coefficient | $t$-value | Significance level ($p$) |
|---|---|---|---|---|
| Constant | 97.5569 | 2.567 | 38.002 | 0.000 |
| Linear | | | | |
| $X_1$ | 5.8195 | 2.043 | 2.849 | 0.036 |
| $X_2$ | -4.2907 | 2.043 | -2.100 | 0.090 |
| Quadratic | | | | |
| $X_1 X_1$ | -3.3130 | 3.144 | -1.054 | 0.340 |
| $X_2 X_2$ | -0.2173 | 3.144 | -0.069 | 0.948 |
| Interaction | | | | |
| $X_1 X_2$ | 3.2461 | 2.502 | 1.297 | 0.251 |
| $r^2$ | | | | 0.755 |

[1]$X_1$, and $X_2$ are the concentration of coating material and the amount of core material, respectively.

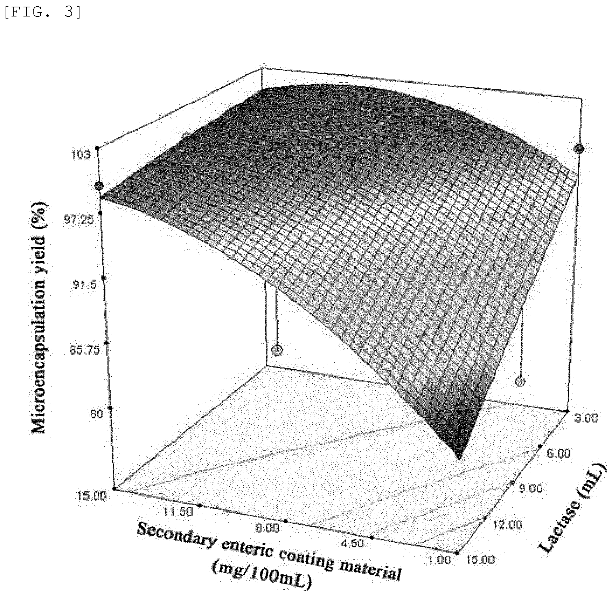

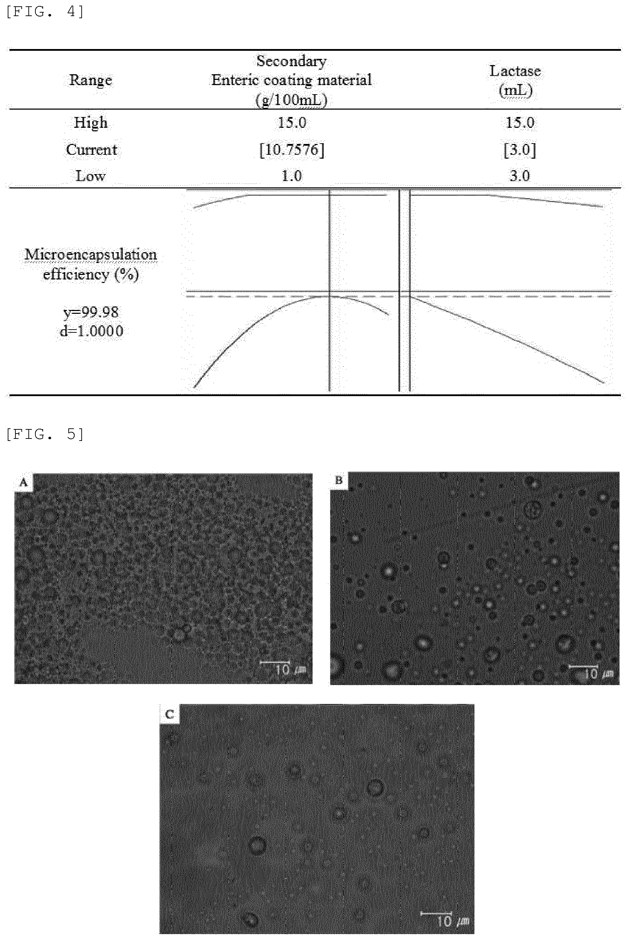

From the results in the above tables, a regression equation for a yield Y of microencapsulation with a change in each independent variable is illustrated in FIGS. 3 and 4. In this case, the $R^2$ value was 0.755.

According to the obtained regression equation, the highest yield of 99.98% was shown when the concentration of the coating material was 10.7576 g/mL and the amount of the core material was 3.0 mL.

3). Microscopic observation of enteric lactase microcapsules

Using the optimized process, the enteric lactase microcapsules coated with various coating materials (HPMCP, shellac, and zein) were observed using an optical microscope (Eclipse 80i, Nikon, Tokyo, Japan) by magnifying the same to 1,000 times.

It could be confirmed that the lactase was captured inside the capsule, and round-shaped capsules were evenly dispersed (FIG. 5).

4). Measurement of zeta potential of enteric lactase microcapsule

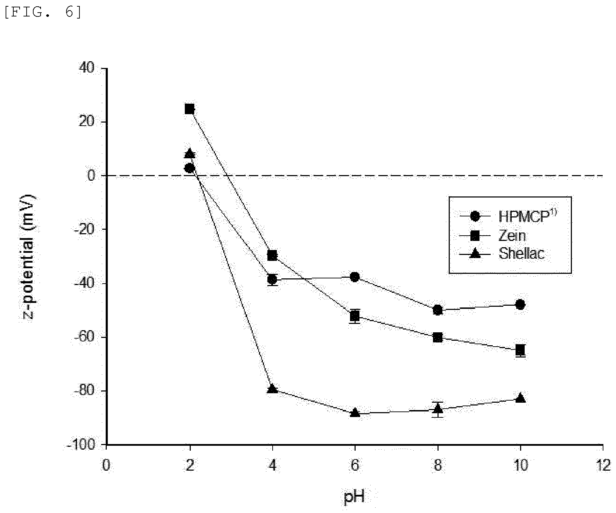

Using the optimized process, zeta potentials of the enteric lactase microcapsules coated with various coating materials (HPMCP, shellac, and zein) were measured. The measured results are illustrated in FIG. 6.

As illustrated in FIG. 6, zeta potentials were measured while continuously changing the pH from 2 to 12. Consequently, as the pH is increased, the absolute value of the zeta potential tended to be increased. In particular, the zeta potentials of the lactase microcapsules coated with HPMCP, shellac, and zein were shown to be -41.2, -59.1, and -90.0 mV, respectively, near pH 6.8 which is a typical pH of milk.

In general, when the zeta potential is ±30 mV or more, it can be said that the dispersibility of the particles is good (Freitas & Muller, Int. J. Pharm., 168, 221-229 (1988)). Based on these results, it can be seen that lactase microcapsules coated with an enteric coating material may be excellent in terms of dispersibility in milk.

In a case of liquid lactase microcapsules coated with MCT and PGMS of Kwak et al. J. Dairy Sci. 84, 1576-1582 (2001), there is a disadvantage that the microcapsules float due to the formation of a W/O emulsion, when adding to the milk. Because of this, there is a disadvantage that an appearance thereof is not good, as well as a fat layer surrounding the lactase is easily acidified by coming into contact with oxygen in the air. On the other hand, the microcapsules of the present invention are excellent in terms of dispersibility in milk, and thus it can be considered that the above disadvantage has been overcome.

5). Size distribution of enteric lactase microcapsules

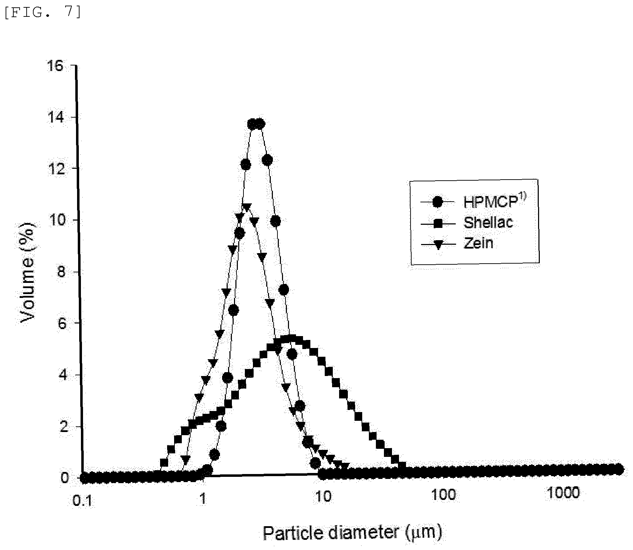

As a result of measuring sizes (particle diameters) of the enteric lactase microcapsules (FIG. 7), the lactase microcapsules coated with HPMCP, shellac, and zein had sizes of 3.370, 5.192, and 2.686 μm, respectively.

Kobayashi et al., Langmuir, 21, 5761-5769 (2005) reported that the smaller the particle diameter, the slower and more stable the aggregation of the particles in the solution. Therefore, based on the above measured results of the zeta potential and the results of the size distribution, it can be seen that the enteric lactase microcapsules of the present invention may be well dispersed in milk.

6). Sensory evaluation

The results of the sensory evaluation for 12 storage days were as illustrated in Table 4 below. As illustrated in Table 4, HPMCP, shellac, and zein showed a result that sweetness thereof was slightly increased during the storage period.

However, HPMCP had no significant difference compared to the initial state, and also had no significant difference as compared to the market milk (control) ($p < 0.05$).

Shellac showed higher sweetness than HPMCP, and there was a significant difference ($p < 0.05$) as compared to the market milk and the initial state from days 8 and 10 of the storage. It was confirmed that zein shown a relatively lower increase in sweetness than the other two samples.

In a case of off-taste, HPMCP and shellac tended to increase slightly during the storage period, and higher off-taste than HPMCP was observed in shellac. Further, it could be seen that both samples showed a significant difference ($p < 0.05$) from day 8 of the storage as compared to the market milk.

However, zein showed a higher value of off-taste than the other samples from the beginning, and had the highest increase in the off-taste over the storage period, that is, zein had a significant difference in the off-taste compared to the control and other samples ($p < 0.05$). Therefore, a result that zein was not preferable in terms of sensory properties can be obtained.

It can be seen that HPMCP and shellac do not show a large difference in terms of sensoryproperties compared to the market milk during the typical consumption period, when considering that the consumption period of the market milk is generally one week or less.

[Table 4]

| Sensory description | Treatment | Storage period (d) | | | | | | |
|---|---|---|---|---|---|---|---|---|
| | | 0 | 2 | 4 | 6 | 8 | 10 | 12 |
| Sweetness | Control[3] | 1.0[aA] | 1.0[aA] | 1.0[aA] | 1.0[aA] | 1.0[bA] | 1.0[bA] | 1.0[bA] |
| | HPMCP | 1.0[aA] | 1.1[aA] | 1.2[aA] | 1.2[aA] | 1.2[abA] | 1.3[abA] | 1.4[abA] |
| | Shellac | 1.0[aC] | 1.1[aBC] | 1.1[aBC] | 1.3[aABC] | 1.4[aABC] | 1.5[aAB] | 1.7[aA] |
| | Zein | 1.0[aA] | 1.0[aA] | 1.0[aA] | 1.0[aA] | 1.1[bA] | 1.1[bA] | 1.1[bA] |
| Off-taste | Control | 1.0[aA] | 1.0[bA] | 1.0[bA] | 1.0[bA] | 1.0[cA] | 1.0[dA] | 1.0[dA] |
| | HPMCP | 1.0[aC] | 1.1[bBC] | 1.3[bBC] | 1.4[bBC] | 1.6[bABC] | 1.7[cAB] | 1.9[cA] |
| | Shellac | 1.0[aD] | 1.3[abCD] | 1.4[bCD] | 1.7[bBC] | 1.9[bB] | 2.3[bA] | 2.6[bA] |
| | Zein | 1.2[aF] | 1.4[aEF] | 1.7[aE] | 2.3[aD] | 3.2[aC] | 3.8[aB] | 4.3[aA] |

[1]HPMCP: hydroxy propyl methyl cellulose phthalate
[2]Values with different superscript in a column (a-d) and raw (A-F) are significant at $p < 0.05$ by Duncan's multiple range test
[3]Control: market milk

7). Evaluation of physicochemical properties of milk added with enteric lactase microcapsule

(1). pH

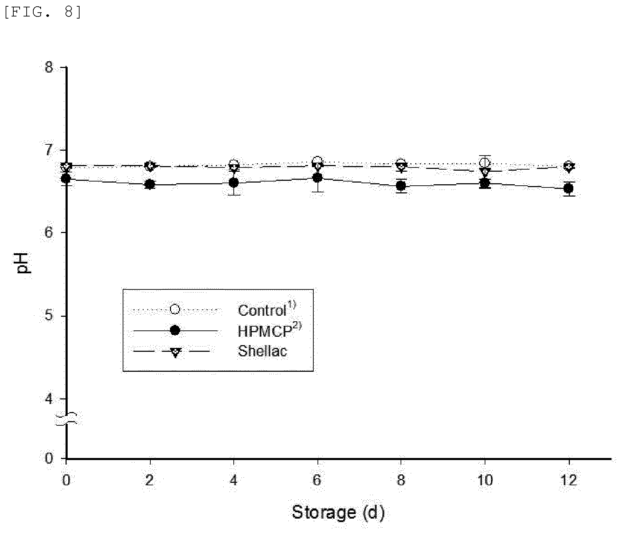

As illustrated in FIG. 8, the market milk without capsule (control), milk (HPMCP) containing capsules coated with HPMCP, and milk (shellac) containing capsules coated with shellac showed a pH of 6.79 to 6.80, 6.65 to 6.53, and 6.81 to 6.80, respectively, and showed no significant change in pH during the storage period.

(2). Chromaticity

Changes in chromaticity during the storage period are stated in Table 5 below. As illustrated in Table 5, shellac had a significantly reduced L* color value to day 4 of the storage, but there was no significant difference thereafter ($p<0.05$). In addition, HPMCP showed no significant difference during the storage period ($p<0.05$).

Meanwhile, HPMCP and shellac showed a slightly higher a* color value than the control, but there was no significant difference ($p<0.05$). Shellac showed a slightly higher b* color value than the control until day 6 of the storage, but there was no significant difference thereafter ($p<0.05$) .

From the above results, it can be seen that the addition of enteric lactase microcapsules does not significantly affect the chromaticity of milk.

[Table 5]

| Color value | Treatment | Storage period (d) | | | | | | |
|---|---|---|---|---|---|---|---|---|
| | | 0 | 2 | 4 | 6 | 8 | 10 | 12 |
| L* | Control[3] | 90.45[aAB] | 90.05[aAB] | 89.96[aB] | 90.09[aAB] | 91.01[aAB] | 90.11[aA] | 90.17[aAB] |
| | HPMCP | 90.44[aA] | 89.99[aA] | 89.83[aA] | 89.89[aA] | 89.46[aA] | 90.05[aA] | 90.14[aA] |
| | Shellac | 88.90[bA] | 89.30[bA] | 89.30[bA] | 89.44[aA] | 89.31[aA] | 89.58[aA] | 89.85[aA] |
| a* | Control | 3.33[aA] | 3.49[aB] | 3.42[aAB] | 3.45[aAB] | 3.41[aAB] | 3.49[aB] | 3.34[aA] |
| | HPMCP | 3.38[aA] | 3.48[aA] | 3.42[aA] | 3.46[aA] | 3.48[aA] | 3.47[aA] | 3.34[aA] |
| | Shellac | 3.69[bB] | 3.60[aAB] | 3.41[aA] | 3.51[aA] | 3.75[bB] | 3.69[aB] | 3.47[aA] |
| b* | Control | 6.06[bAB] | 5.84[bC] | 6.06[abAB] | 5.95[aBC] | 6.18[aA] | 5.95[aBC] | 6.00[aB] |
| | HPMCP | 6.03[bB] | 5.78[bC] | 5.92[bBC] | 5.92[aBC] | 6.28[aA] | 5.80[bC] | 5.91[aBC] |
| | Shellac | 6.56[aA] | 6.38[aAB] | 6.28[aAB] | 6.00[aBC] | 6.57[aA] | 5.82[bC] | 6.40[aAB] |

[1]HPMCP: hydroxy propyl methyl cellulose phthalate

[2]Values with different superscript in a column (a-c) and raw (A-G) are significant at $p<0.05$ by Duncan's multiple range test

[3]Control: market milk

(3). Stability of lactase microcapsule

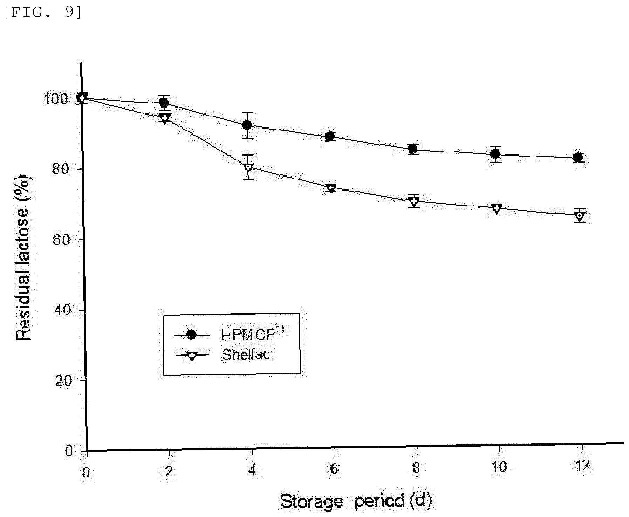

The stability of lactase microcapsules was observed by measuring the residual lactose during the storage. As a result, both HPMCP and shellac showed a gradual decrease in the residual lactose over time (FIG. 9).

On day 12 of the storage, HPMCP and shellac showed 81.18% and 64.91% of the residual lactose, respectively, such that the HPMCP was more stable in milk than the shellac. These are much better results than a case in which the residual lactose in milk added with liquid lactase microcapsules coated with PGMS and MCT is 28.81% at day 12 of the storage, which is reported by Kwak et al. J. Dairy Sci. 84, 1576-1582 (2001).

8). Evaluation of *in vitro* release characteristics of enteric lactase microcapsule

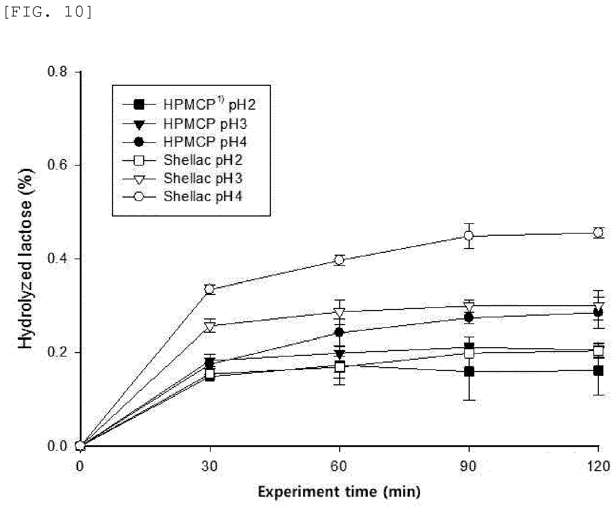

As foods enter the stomach and are mixed with gastric acid, the pH is gradually decreased. In order to illustrate this phenomenon, the release rates at pH 2, 3, and 4 were observed, respectively. Results thereof are illustrated in FIG. 10.

As illustrated in FIG. 10, HPMCP and shellac showed release rates of 0.1 and 0.2%, respectively, at pH 2.0 for 120 minutes, and it could be seen that they effectively protected lactase, which is a core material, with releasing minimal amount in a gastrointestinal environment of an acidic condition.

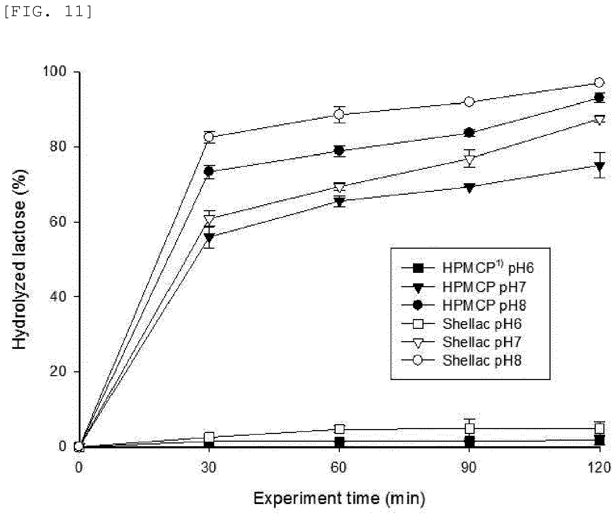

By contrast, in an artificial small intestine environment, HPMCP and shellac showed release rates of 75, 87%, and 93, 97% at pH 7 and 8 for 120 minutes, respectively. Therefore, it could be seen that the enteric lactase microcapsules could effectively release the core material in a short time in the small intestine environment, thereby helping to improve the lactose intolerance (FIG. 11) .

9). Clinical experiment

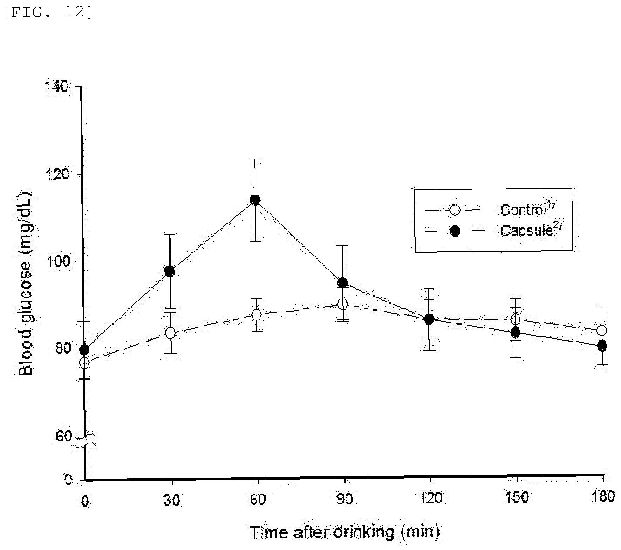

Persons having lactose intolerance drunk milk mixed with enteric-coated lactase microcapsules on an empty stomach, then blood glucoses thereof were measured every 30 minutes for 3 hours. The measured results are illustrated in FIG. 12.

As illustrated in FIG. 12, it was observed that blood glucose is rapidly increased up to 60 minutes after drinking, compared to the case of drinking the general market milk (control). This means that the milk is decomposed by the lactase released from the enteric lactase microcapsules into monosaccharides in the small intestine and absorbed into the body to be used as an energy source, thereby improving the lactose intolerance.

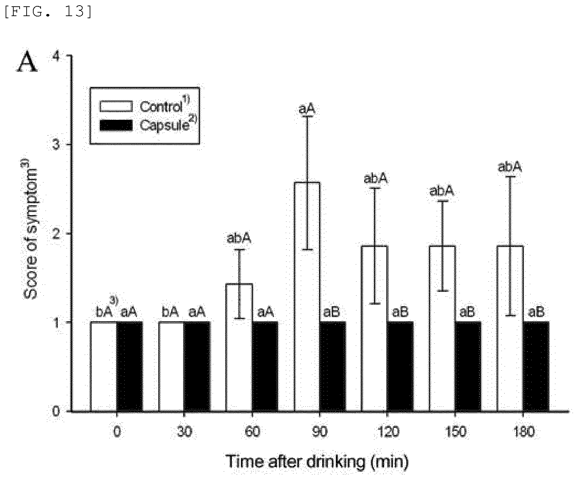

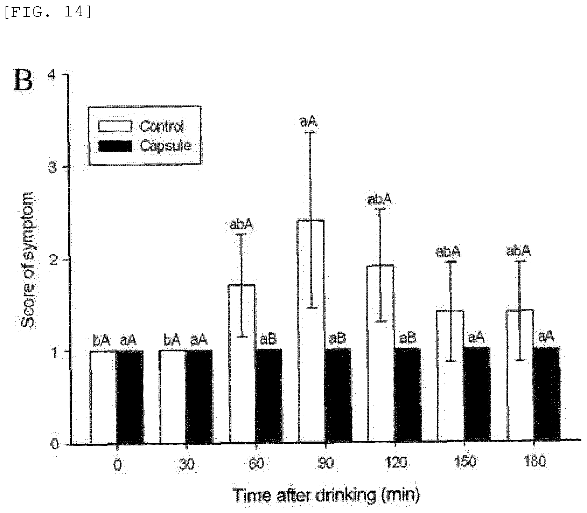

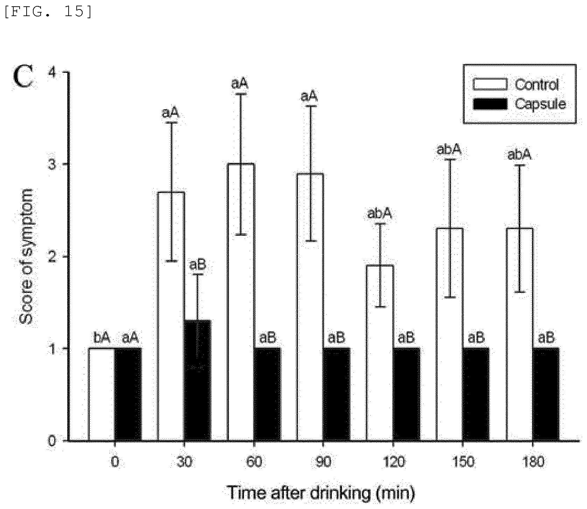

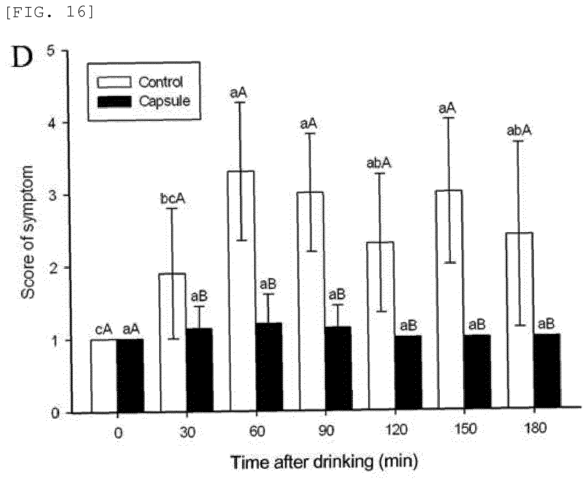

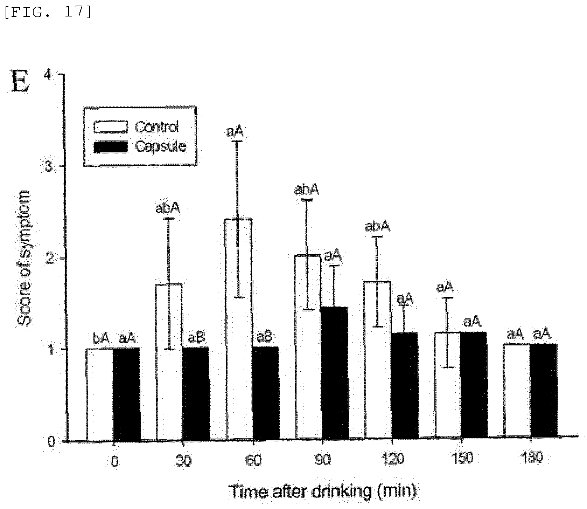

During the test of blood glucose, intensities of feeling diarrhea, abdominal pain, abdominal bloating, audible bowel sound, nausea, and the like, which are typically known symptoms caused by lactose intolerance were recorded by the test persons using the 5-score method (1 = very weak, 3 = moderate, 5 = very strong). The evaluated results are illustrated in FIGS. 13 to 17.

In FIGS. 12 to 17, the market milk product is used as the control.

Referring to FIGS. 13 to 17, it was confirmed that most of the symptoms after drinking milk added with microcapsules were significantly reduced compared to the case of drinking the market milk, and it could be seen that milk added with enteric lactase microcapsules effectively improved the lactose intolerance.

<Example 1>

An emulsion of lactase was obtained by providing lactase as a core material, and stirring the core material with medium-chain triglyceride (MCT) as a primary coating material and polyglycerol polyricinoleate (PGPR) having an HLB value of 0.6, and in a concentration of 1.0% as a primary emulsifier at 60°C and 1,500 rpm for 30 minutes to obtain a pre-emulsion, followed by stirring the same at 60°C and 9,000 rpm for 2 minutes.

At this time, the core material and the primary coating material were mixed in a ratio of 1:3 (v/v), and the core material and the primary emulsifier were mixed in a ratio of 1:3 (v/v), followed by stirring the same.

Then, a double microcapsule emulsion of lactase was obtained by providing the emulsion of lactase as a core material, and stirring the emulsion of lactase with HPMCP (Samsung Fine Chemicals Co., Ltd.) as a secondary coating material and PSML having an HLB value of 16.7, and in a concentration of 1.0% at 60°C and 2,000 rpm for 10 minutes as a secondary emulsifier to homogenize the same, followed by centrifuging at 4°C and 3,500 xg for 15 minutes.

At this time, the secondary coating material was used by dissolving in 85% (v/v) ethanol. The emulsion of lactase and the secondary coating material were mixed in a ratio of 2.5:2.75 (v/v), and the emulsion of lactase and the secondary emulsifier were mixed in a ratio of 2.5:2.75 (v/v), followed by stirring and centrifuging the same.

Finally, lactase-containing double microcapsules were prepared by pulverizing the obtained double microcapsule emulsion of lactase by spray drying.

At this time, Godo YNL-2 (Godo Shusei Co., Ltd., Tokyo, Japan) was used as the lactase.

<Example 2>

Lactase-containing double microcapsules were prepared according to the same procedure as described in Example 1, except that shellac (Shellac Korea Co., Ltd.) was used instead of HPMCP as the secondary coating material.

<Example 3>

Lactase-containing double microcapsules were prepared according to the same procedure as described in Example 1, except that hydrogenated corn oil was used instead of MCT as the primary coating material and zein (Pooglimmuyark Co., Ltd.) was used instead of HPMCP as the secondary coating material.

<Example 4>

Lactase-containing double microcapsules were prepared according to the same procedure as described in Example 1, except that soybean oil was used instead of MCT as the primary coating material and shellac (Shellac Korea Co., Ltd.) was used instead of HPMCP as the secondary coating material.

<Example 5>

Lactase-containing double microcapsules were prepared according to the same procedure as described in Example 1, except that safflower seed oil was used instead of MCT as the primary coating material and zein (Pooglimmuyark Co., Ltd.) was used instead of HPMCP as the secondary coating material.

<Example 6>

An emulsion of lactase was obtained by providing a mixture in which lactase and a functional component are mixed in a ratio of 1:1 (v/v) as a core material, and stirring the core material with MCT as a primary coating material and polyglycerol polyricinoleate (PGPR) having an HLB value of 0.6, and in a concentration of 1.0% as a primary emulsifier at 60°C and 1,500 rpm for 30 minutes to obtain a pre-emulsion, followed by stirring the same at 60°C and 9,000 rpm for 2 minutes.

At this time, the core material and the primary coating material were mixed in a ratio of 1:3 (v/v), and the core material and the primary emulsifier were mixed in a ratio of 1:3 (v/v), followed by stirring the same.

Then, a double microcapsule emulsion of lactase was obtained by providing the emulsion of lactase as a core material, and stirring the emulsion of lactase with HPMCP (Samsung Fine Chemicals Co., Ltd.) as a secondary coating material and polyoxyethylene sorbitan monolaurate (PSML) having an HLB value of 16.7, and in a concentration of 1.0% at 60°C and 2,000 rpm for 10 minutes as a secondary emulsifier to homogenize the same, followed by centrifuging at 4°C and 3,500 xg for 15 minutes.

The secondary coating material was used by dissolving in 85% (v/v) ethanol. The emulsion of lactase and the secondary coating material were mixed in a ratio of 2.5:2.75 (v/v), and the emulsion of lactase and the secondary emulsifier were mixed in a ratio of 2.5:2.75 (v/v), followed by stirring and centrifuging the same.

Finally, double microcapsules including the lactase and functional component were prepared by pulverizing the obtained double microcapsule emulsion of lactase by spray drying.

In the above example, Godo YNL-2 (Godo Shusei Co., Ltd., Tokyo, Japan) was used as the lactase, and Juniperus rigida fruit extract, which is obtained by: putting and mixing Juniperus rigida fruit, which is a fruit extract of Juniperus rigida S. et Z, and is washed and dried after removing foreign matters, in purified water of 10 times based on a weight of the Juniperus rigida fruit; then extracting and filtering the same so as to have a volume of 25% based on a volume of initial purified water at a temperature of 100°C to obtain a primary filtrate; and decompression concentrating the primary filtrate, followed by vacuum freeze drying so as to have a volume of 30% based on a volume of the primary filtrate, was used as the functional component.

<Application Example 1>

Milk including lactase-containing double microcapsules was produced by adding 5% by weight of lactase-containing double microcapsules prepared in Example 1 to milk.

<Application Example 2>

Milk including lactase-containing double microcapsules was produced by adding 5% by weight of lactase-containing double microcapsules prepared in Example 2 to milk.

<Application Example 3>

Milk including lactase-containing double microcapsules was produced by adding 5% by weight of lactase-containing double microcapsules prepared in Example 3 to milk.

<Application Example 4>

Milk including lactase-containing double microcapsules was produced by adding 5% by weight of lactase-containing double microcapsules prepared in Example 6 to milk.

<Application Example 5>

Milk including lactase-containing double microcapsules was produced by adding 1% by weight of lactase-containing double microcapsules prepared in Example 1 to milk.

<Application Example 6>

Milk including lactase-containing double microcapsules was produced by adding 10% by weight of lactase-containing double microcapsules prepared in Example 1 to milk.

<Application Example 7>

Yogurt including lactase-containing double microcapsules was produced by adding 5% by weight of lactase-containing double microcapsules prepared in Example 1 to yogurt.

<Application Example 8>

Goat milk yogurt including lactase-containing double microcapsules was produced by adding 5% by weight of lactase-containing double microcapsules prepared in Example 6 to goat milk yogurt.

<Application Example 9>

Healthy supplements of whey protein concentrates including lactase-containing double microcapsules was produced by adding 5% by weight of lactase-containing double microcapsules prepared in Example 1 to whey protein concentrates.

<Application Example 10>

An ice cream including lactase-containing double microcapsules was prepared using milk including the lactase-containing double microcapsules prepared in Application Example 1.

While the present invention has been described with reference to the preferred embodiments, examples and application examples, the present invention is not limited thereto, and it will be understood by those skilled in the art that various modifications and variations may be made within the detailed description of the invention and accompanying drawings without departing from the scope of the present invention as defined by the appended claims.

[Industrial Applicability]

Since the lactase-containing double microcapsule according to the present invention uses an enteric coating material which is dissolved only in the small intestine, it is possible to greatly improve not only the advantage of the microencapsulation but also the stability in the milk, as well as solve the lactose intolerance of dairy products. Therefore, the present invention can be applied to a technical field to which the present invention pertains.

**Claims**

1. A lactase-containing double microcapsule comprising: lactase provided as a core material; and a primary coating material and a secondary coating material, which are sequentially coated on the core material to be formulated.

2. The lactase-containing double microcapsule according to claim 1, wherein the primary coating material includes at least one selected from the group consisting of medium-chain triglyceride (MCT), hydrogenated corn oil, soybean oil, safflower seed oil and butter oil.

3. The lactase-containing double microcapsule according to claim 1, wherein the secondary coating material includes at least one selected from the group consisting of hydroxypropyl methylcellulose phthalate, zein, shellac, Eudragit, cellulose acetate phthalate, cellulose acetate succinate, polyvinyl acetate phthalate, cellulose acetate trimellitate, hypromellose acetate succinate and phenyl salicylate.

4. The lactase-containing double microcapsule according to claim 1, wherein the formulation is in any form selected from the group consisting of powders, solution, tablets, and granules.

5. A method for preparing lactase-containing double microcapsule comprising:

   providing lactase as a core material, and stirring the core material with a primary coating material and a primary emulsifier to obtain an emulsion of lactase;
   stirring the emulsion of lactase with a secondary coating material and a secondary emulsifier dissolved in alcohol to obtain a double microcapsule emulsion of lactase; and
   formulating the obtained double microcapsule emulsion of lactase.

6. The method for preparing lactase-containing double microcapsule according to claim 5, wherein the lactase is derived from a strain selected from the group consisting of *Kluyveromyces lactis, Aspergillus oryzae, Aspergillus niger, Bacillus circulans, Escherichia coli* and *Bos Taurus,* oris recombinant lactase.

7. The method for preparing lactase-containing double microcapsule according to claim 5, comprising:

providing lactase as a core material, and stirring the core material with at least one selected from the group consisting of medium-chain triglyceride (MCT), hydrogenated corn oil, soybean oil, safflower seed oil and butter oil as a primary coating material and at least one selected from the group consisting of polyglycerol polyricinoleate, sucrose fatty acid ester, and polyglycerol fatty acid ester as a primary emulsifier to obtain a pre-emulsion, and further stirring the mixture to obtain an emulsion of lactase,
wherein the core material and the primary coating material are mixed in a ratio of 1:9 (v/v) to 9:1 (v/v) , and the core material and the primary emulsifier are mixed in a ratio of 1:9 (v/v) to 9:1 (v/v), and then stirred to obtain an emulsion of lactase;
providing the emulsion of lactase as a core material, adding any one selected from the group consisting of hydroxypropyl methylcellulose phthalate, zein, shellac, Eudragit, cellulose acetate phthalate, cellulose acetate succinate, polyvinyl acetate phthalate, cellulose acetate trimellitate, hypromellose acetate succinate and phenyl salicylate as a secondary coating material to the obtained emulsion of lactase, and stirring to homogenize the same, followed by centrifuging to obtain a double microcapsule emulsion of lactase, wherein the emulsion of lactase and the secondary coating material are mixed in a ratio of 1:9 (v/v) to 9:1 (v/v), and the emulsion of lactase and the secondary emulsifier are mixed in a ratio of 1:9 (v/v) to 9:1 (v/v), and then stirred, followed by centrifuging to obtain a double microcapsule emulsion of lactase; and
formulating the obtained double microcapsule emulsion of lactase.

8. The method for preparing lactase-containing double microcapsule according to claim 5, wherein the alcohol is an alcohol having 1 to 4 carbon atoms.

9. The method for preparing lactase-containing double microcapsule according to claim 5, wherein the formulation is in any form selected from the group consisting of powders, solution, tablets, and granules.

10. A dairy product comprising the lactase-containing double microcapsule according to any one of claims 1 to 4.

11. The dairy product according to claim 10, wherein the dairy product is any one selected from the group consisting of milk, yogurt, goat milk, whey protein concentrates and ice cream.

[FIG. 1]

Dispersion fluid

Emulsion

[FIG. 2]

[FIG. 3]

[FIG. 4]

| Range | Secondary Enteric coating material (g/100mL) | Lactase (mL) |
|---|---|---|
| High | 15.0 | 15.0 |
| Current | [10.7576] | [3.0] |
| Low | 1.0 | 3.0 |

Microencapsulation efficiency (%)

y=99.98
d=1.0000

[FIG. 5]

[FIG. 6]

[FIG. 7]

[FIG. 8]

[FIG. 9]

[FIG. 10]

[FIG. 11]

[FIG. 12]

[FIG. 13]

[FIG. 14]

[FIG. 15]

[FIG. 16]

[FIG. 17]

<div align="center">**INTERNATIONAL SEARCH REPORT**</div>

| | International application No. |
|---|---|
| | **PCT/KR2016/010474** |

| A. CLASSIFICATION OF SUBJECT MATTER |
|---|
| *A23C 9/12(2006.01)i, A23L 29/00(2016.01)i, A23P 10/35(2016.01)i, A23P 10/20(2016.01)i, A23P 10/40(2016.01)i* |
| According to International Patent Classification (IPC) or to both national classification and IPC |

| B. FIELDS SEARCHED |
|---|
| Minimum documentation searched (classification system followed by classification symbols) |
| A23C 9/12; A23L 19/00; A61K 9/48; A23C 9/152; A61K 35/74; A23K 1/00; A23C 9/123; A23K 1/16; A23L 29/00; A23P 10/35; A23P 10/20; A23P 10/40 |
| Documentation searched other than minimum documentation to the extent that such documents are included in the fields searched<br>Korean Utility models and applications for Utility models: IPC as above<br>Japanese Utility models and applications for Utility models: IPC as above |
| Electronic data base consulted during the international search (name of data base and, where practicable, search terms used)<br>eKOMPASS (KIPO internal) & Keywords: double microcapsule, lactase, first coating material, second coating material |

| C. DOCUMENTS CONSIDERED TO BE RELEVANT | | |
|---|---|---|
| Category* | Citation of document, with indication, where appropriate, of the relevant passages | Relevant to claim No. |
| Y | KR 10-1999-0070766 A (KWAK, Hae Soo) 15 September 1999<br>See claim 1. | 1-11 |
| Y | KR 10-2013-0055236 A (INDUSTRY ACADEMY COOPERATION FOUNDATION OF SEJONG UNIVERSITY et al.) 28 May 2013<br>See paragraph [0027] and claims 1-3, 5-6. | 1-11 |
| Y | KR 10-2013-0070111 A (HANMI PHARM. CO., LTD.) 27 June 2013<br>See claim 14. | 3,7 |
| A | KR 10-2003-0070799 A (KOOK SOON DANG CO., LTD. et al.) 02 September 2003<br>See claims 1-2, 9-10. | 1-11 |
| A | KR 10-0869900 B1 (REPUBLIC OF KOREA(MANAGEMENT : RURAL DEVELOPMENT ADMINISTRATION) et al.) 24 November 2008<br>See claims 1, 8. | 1-11 |

| ☐ Further documents are listed in the continuation of Box C. | ☒ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 15 MAY 2017 (15.05.2017) | **15 MAY 2017 (15.05.2017)** |
| Name and mailing address of the ISA/KR<br>Korean Intellectual Property Office<br>Government Complex-Daejeon, 189 Seonsa-ro, Daejeon 302-701, Republic of Korea<br>Facsimile No. +82-42-481-8578 | Authorized officer<br><br><br>Telephone No. |

Form PCT/ISA/210 (second sheet) (January 2015)

INTERNATIONAL SEARCH REPORT
Information on patent family members

International application No.

**PCT/KR2016/010474**

| Patent document cited in search report | Publication date | Patent family member | Publication date |
|---|---|---|---|
| KR 10-1999-0070766 A | 15/09/1999 | KR 10-0260592 B1<br>US 6491955 B1 | 01/07/2000<br>10/12/2002 |
| KR 10-2013-0055236 A | 28/05/2013 | KR 10-1355488 B1 | 29/01/2014 |
| KR 10-2013-0070111 A | 27/06/2013 | KR 10-1378974 B1 | 31/03/2014 |
| KR 10-2003-0070799 A | 02/09/2003 | NONE | |
| KR 10-0869900 B1 | 24/11/2008 | KR 10-2008-0036311 A | 28/04/2008 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- KR 1019930022957
- KR 1019930022958

### Non-patent literature cited in the description

- **KWAK et al.** *Int. Dairy J.,* 2012, vol. 22, 147-151
- **KAWAKTSU et al.** *Colloids Surf. A,* 2001, vol. 189, 257-264
- **LUAN et al.** *Int. J. Pharm.,* 2006, vol. 324, 168-175
- **PAPAGIANNI et al.** *Enzyme Microb. Technol.,* 2009, vol. 45, 514-552
- **KWAK ; JEON.** *J. Food Sci.,* 1988, vol. 53, 975-976
- **FINKENSTEDT et al.** *Brit. med. J.,* 1986, vol. 292, 161-162
- **GREENBERGER ; ISSELBACHER.** harrison's principles of internal medicine. McGraw-Hill, 1983, 1735
- **FREITAS ; MULLER.** *Int. J. Pharm.,* 1988, vol. 168, 221-229
- **KWAK et al.** *J. Dairy Sci.,* 2001, vol. 84, 1576-1582
- **KOBAYASHI et al.** *Langmuir,* 2005, vol. 21, 5761-5769